# EUROPEAN PATENT SPECIFICATION

(11) **EP 2 923 287 B1**
(45) Date of publication and mention of the grant of the patent: **27.09.2017**
(21) Application number: 13796150.4
(22) Date of filing: 22.11.2013
(51) Int. Cl.: G06F 17/50, A61F 5/14, A43B 17/03, B33Y 80/00, A43B 7/14, B33Y 50/00

(54) **ORTHOSIS**
ORTHESE
ORTHÈSE

(30) Priority: 23.11.2012 GB 201221135
(43) Date of publication of application: 30.09.2015
(73) Proprietor: Kent Community Health NHS Foundation Trust, Barming Maidstone, Kent ME16 9NT (GB)
(72) Inventor: OLK, Jatinder, Ashford Kent TN25 4AZ (GB); BELLMAN, Cathy, Ashford Kent TN25 4AZ (GB); FRIPP, Tom, Sheffield South Yorkshire S60 5WG (GB); GREEN, Lewis, Sheffield South Yorkshire S60 5WG (GB)
(74) Representative: Foot, Paul Matthew James
(86) International application number: PCT/GB2013/053091
(87) International publication number: WO 2014/080217

(56) References cited:
- EP-A2- 1 116 449
- DE-U1-202004 017 280
- US-A1- 2007 022 630
- US-A1- 2010 262 054
- US-B1- 6 681 501

## Description

### FIELD OF THE INVENTION

The present invention relates to a method of manufacturing an orthosis and/or an orthosis.

### BACKGROUND OF THE INVENTION

An orthosis is a device used to support and/or modify a muscular or skeletal system. There are many types of orthoses, including foot othoses, ankle-foot orthoses, knee-ankle-foot orthoses, knee orthoses, upper limb orthoses, hand orthoses, and spinal orthoses. A foot orthosis generally takes the form of an insole for a shoe. The insole has a region of support and/or relief. The region of support may be a profiled region designed to support the foot and provide correct positioning of the foot. The region of relief may be a soft/cushioned region aimed to reduce a force applied to a specific region of a patient's foot so as to relieve pain. A foot orthosis may be used to support a foot post surgery, to improve gait of a user, and/or to provide relief from conditions such as pressure ulcers. The current method of manufacturing a foot orthosis includes taking a model of a patient's foot, e.g. by taking a casting or a scan of a patient's foot. The design of the orthosis is then developed using manual techniques and sometimes with the aid of software (for example using Delcam OrthoModel, provided by Delcam plc, Small Heath Business Park, Birmingham, B10 0HJ, UK).

The foot orthosis is then manufactured from a block of material by machining. Soft regions may be machined as a recess in the block of material and a soft material positioned in the recess and attached using a form of adhesive.The current method of manufacturing orthoses is labour intensive and time consuming.

Examples of known methods of manufacturing orthoses are disclosed in US2010/262054, US2007/022630 and DE202004017280. US2010/262054 discloses a shell of a brace that is formed in a process, where softer pads are secured to the shell in a second, separate process. US2007/022630 discloses an insole for footwear having regions/components of different hardnesses. DE202004017280 discloses an insole for an orthopaedic shoe, which is produced by milling a solid multi-layer block where each layer has a different hardness.

### SUMMARY OF THE INVENTION

The present invention seeks to alleviate one or more problems associated with the prior art.

A first aspect of the invention provides a method of manufacturing an orthosis comprising: providing electronic 3D data defining a shape of an orthosis and a first region and a second region of the orthosis, the first region having a greater compressibility than the second region; and using a 3D printer to print an orthosis based on the electronic 3D data, wherein the 3D printer selectively uses materials of two or more shore hardnesses to print the orthosis to provide varying hardness across the orthosis, said materials being printed together in a single 3D printing process, such that the first region is integrally formed with the second region.

The present inventors have taken the inventive step of looking outside the technologies known to the skilled clinician and orthosis designer and have realised that 3D printing can be used to form an orthosis. 3D printing permits the time, cost and labour intensiveness of the manufacture of the orthosis to be reduced when compared to conventional manufacturing methods. 3D printing also negates the need for finishing operations such as grinding. Further, utilising 3D printing enables greater design freedom and customisation enabling features to be added to the orthosis that would be difficult or impossible to achieve using conventional machining methods. Use of two or more materials of different shore hardness permits the orthosis to have the required variation of material properties across the orthosis without the need for attachment of two or more sections of the orthosis, for example without the need for an adhesive.

In the present application the orthosis manufactured may be a complete orthosis or may form part of a complete orthosis. For example, the method of manufacture may be used to form a shell of an orthosis and components (e.g. a cushioned pad) may be added to the shell to form a complete orthosis. Two or more materials (e.g. two, three or four materials) of different shore hardnesses may be mixed in varying quantities to provide varying shore hardness across the orthosis. The second region may be formed using a material of greater shore hardness than the first region.

The first and/or second region may comprise geometric grading for a greater compressibility in the first region than in the second region. Geometric grading permits the orthosis to have the required variation of material properties across the orthosis without the need for attachment of two or more sections of the orthosis, for example without the need for an adhesive. Further, when the method uses both materials of two or more shore hardnesses and geometric grading, the orthosis can be further optimised for a user's needs. Many geometric grading structures suitable for providing different levels of compressibility in an orthosis would not be possible or would be very difficult to manufacture using the conventional machining methods of the prior art.

Geometric grading refers to modifying the construction of a region to modify the compressibility of that region, taking into account the hardness of material in said region. The geometric grading may utilise one or more of, but not exclusively, dome shapes, wave shapes, pillars, columns, tensioned surfaces, cylinders, ribs and/or strips/elongate sections of material. For example, the first region may include a surface structure connected to the remainder of the orthosis. A region below the surface structure may be hollow. This particular construction has been found to be particularly advantageous when used as an orthosis to which relatively large amounts of pressure are applied (for example a foot orthosis), because having no support structures beneath the surface means that there is no risk of a patient feeling the support structures through the surface, which increases comfort for a user.

The 3D data may define a transition region between the first region and the second region. The transition region may have graduated compressibility, e.g. decreasing compressibility from a position in the transition region nearest the first region to a position in the transition region nearest the second region. Graduation may be achieved using a varying mix of materials of different shore hardness, and/or graduation may be achieved using geometric grading (e.g. constructing a series of linear strips of material or suitable patterns of materials of varying hardness).

The method may comprise forming an orthosis with a layered structure, each layer having a different shore hardness. For example, in the case of a foot orthosis, a lower layer intended to be nearest a sole of a shoe may have an increased hardness compared to an upper layer intended to be nearest to a foot of a user.

The method may comprise forming an indentation pattern on a side of the orthosis that is intended to be positioned furthest from the body of a user in use. The indentation pattern can reduce weight of the orthosis, and reduce the amount of printer material used which can reduce the overall unit cost. The indentation pattern may comprise a grid pattern, for example a square or rectangular grid pattern. Alternatively, the indentation pattern may comprise a series of ribs (e.g. wavy or straight). Surprisingly, it was also found that in some cases when the orthosis was a foot orthosis the indentation pattern improves comfort for a user because of the cushioning provided by the air pockets formed between the orthosis and a surface upon which a user's foot and orthosis is placed. In the case where the indentation pattern comprises ribs, the ribs may extend in the direction of heel to toe (of a foot of a user, when in use) or in a direction transverse to the heel to toe direction to add to or reduce the flexibility of the orthosis.

The orthosis may comprise further regions of varying compressibility, for example a third, fourth, fifth and/or sixth region, and two or more of said regions having a different compressibility.

The method may comprise printing multiple orthoses in a single printing process. For example, the multiple orthoses may be printed side by side in a single printing process.

The method may comprise building a CAD model from which at least a portion of the 3D data is derived. In such embodiments, the method may comprise using haptic software to modify the CAD model so as to smooth transitions between regions of a modelled orthosis, and/or add or remove features to or from the orthosis.

The method may comprise scanning a body part against which the orthosis is to be positioned. The method may comprise extracting dimensions from the scanned body part to form the CAD image of the orthosis. Alternatively, the 3D data may be derived from an image and/or data set within a stock library. For example, in the case of a foot orthosis, an image of an orthosis may be selected from an image library, the orthosis selected being one that closely resembles an orthosis suitable for the shape of an intended user's foot and, when used for a medical application, suitable for treating a user's condition.

Software may be used to form an outline of an orthosis compatible with the scanned body part. In such embodiments, human intervention may also be used to use expert knowledge to check that the design orthosis is suitable.

The orthosis may be printed with a gloss finish and/or a textured finish.

The orthosis may be a foot orthosis.

The method may comprise using the 3D printer to add identification details to the orthosis. Adding identification details can reduce errors and allow traceability. For example, when the orthosis is a foot orthosis, the identification details may be printed during manufacture on a side of the orthosis intended to be furthest from the foot of a user.

In a second aspect the present invention provides an orthosis manufactured using the method according to the first aspect.

In a third aspect the present invention provides an orthosis comprising a first region and a second region, the first region having greater compressibility than the second region, and wherein the first region is integrally formed with the second region, wherein one or more materials of the first region have a different shore hardness to one or more materials of the second region, said materials being printed together in a single 3D printing process.

Integrally forming the first and second regions means that no attachment means are required, for example no adhesive is required to adjoin the first region to the second region.

The orthosis of the third aspect may be formed using the method of the first aspect.

An example not forming part of the invention provides an orthosis comprising a first region and a second region, the first region having greater compressibility than the second region, wherein the greater flexibility is provided by geometric grading.

Using geometric grading to form different levels of compressibility in an orthosis can provide increased design options and flexibility to meet a user's needs. For example, the orthosis may be formed using the method of the first aspect.

One or more materials of the first region may have a different shore hardness to one or more materials of the second region.

A transition region may be provided between the first and second region. The transition region may have graduated compressibility, e.g. decreasing compressibility from a position in the transition region nearest the first region to a position in the transition region nearest the second region. Graduation may be achieved using a varying mix of materials of different shore hardness, and/or graduation may be achieved using geometric grading (e.g. constructing a series of linear strips of material or suitable patterns of materials of varying hardness).

The orthosis may be provided as a single integrally formed component. Alternatively, the first and/or second region may be formed as replaceable region(s). Forming the first and/or second regions as replaceable region(s) permits the orthosis to be adjusted over time if required, which means that the life of the orthosis may be extended.

The geometric grading may comprise one or more of pillars, columns, elongate sections, domes, support surfaces, tensioned surfaces, ribs, cylinders and/or cut-outs.

The orthosis may be a foot orthosis.

The orthosis may be shaped for insertion into a heeled shoe or other type of shoe. It has been found that patients are reluctant to change their footwear to a type that is suitable for use with a conventional design of foot orthosis. The provision of an orthosis that can be designed for use in a specific type of shoe, for example a heeled shoe (e.g. a court shoe), hiking boots, football boots, or other type of sports, fashion or casual shoe, means that a user is more likely to use the orthosis, which when used to treat a medical condition can reduce the recovery time of a user.

The orthosis may be configured to form an entire sole of a shoe, e.g. a sole unit. For example, the orthosis may form the sole of a sandal (e.g. flip flop). The sole unit may be configured for attachment to an upper portion of a sandal, or the sole unit may be connected to an upper portion of a sandal (e.g. to fastening straps). For example, the sole may be attached to a foot prong to form a flip-flop, or other light weight footwear.

The orthosis may comprise ventilation holes through some or all of the thickness of the orthosis.

The foot orthosis may comprise a central core region that has a higher shore hardness than an edge region of the orthosis. Such a structure can improve the strength of the main body without compromising flexibility at the edges of the foot orthosis, which contributes to improved comfort for a user.

The foot orthosis may comprise an arch support. The arch support may be made of a harder material than a further region of the foot orthosis. The foot orthosis may comprise one or more impact pads configured for supporting the ball or the heel of the foot. The foot orthosis may comprise a cushion pad for providing relief to a region of a foot, for example for providing relief in the region of a pressure ulcer of a user's foot.

### BRIEF DESCRIPTION OF THE DRAWINGS

Embodiments of the present invention will now be described with reference to the accompanying drawings, in which:
Figure 1 shows a flow diagram of the steps in a method of forming an orthosis according to an embodiment of the present invention;
Figure 2 shows a plan view of an upperside of an orthosis manufactured using the method of Figure 1;
Figure 3 shows a perspective view of the orthosis of Figure 2;
Figure 4 shows a cross sectional view of the orthosis shown in Figures 2 and 3 along the line X-X shown in Figure 2;
Figure 5 shows a plan view of an underside of the orthosis of Figure 2;
Figure 6 shows segments of the underside of Figure 5 as modelled during the manufacturing process of Figure 1;
Figures 7 to 10 show various segments of the upperside of Figure 2 as modelled during the manufacturing process;
Figure 11 shows some segments of the upperside layered on the segments of the underside;
Figure 12 shows a perspective view of a portion of an alternative orthosis manufactured using the method of Figure 1; and
Figure 13 shows a cross sectional view through a section of the orthosis shown in Figures 12 along the line Z-Z shown in Figure 12.

### DETAILED DESCRIPTION OF EMBODIMENT(S)

Orthoses can be used on many parts of the body. Types of orthoses include, by way of example only, hand orthoses, or parts of a neck brace, or a knee brace, or other types of lower limb orthotic, such as night splints, or surgical shoe components.

The following description describes the manufacture and the features of a foot orthosis. However, a similar method could be used to form any orthosis, which can also be designed to have similar features to the foot orthosis herein described.

Foot Orthoses can be used in multiple applications, including but not limited to:
- Optimisation of function - Either for sports or to compensate for congenital or acquired abnormality. For example, to improve gait in a child with Down's syndrome.
- Prevention of pathology - Orthoses are sometimes used to prevent either a structural change or a specific pathology. For example, to reduce the internal compression in the big toe joint to reduce the risk of degeneration.
- Treatment of specific, active pathology - Orthoses can be used to treat Musculoskeletal pathologies both in the feet and higher up, for example to treat compressive knee osteoarthritis.
- Treatment of local manifestations of systemic conditions - Orthoses can be used to limit the impact of global conditions such as Rheumatoid Arthritis.

Foot orthoses may be used to treat numerous conditions. The type of treatment required dictates the form of the foot orthosis required. Conditions and potential treatments include but are not limited to:
- Compressive Medial Knee Osteoarthritis. May be treated using an orthotic with a fairly low arch and a full length lateral wedge.
- Plantar Fasciitis. May be treated using an orthotic with a higher arch and a medial rearfoot wedge.
- Mortons neuroma. May be treated using a softer orthotic with a dome under the ball of the foot.
- Child with Down's syndrome and hypermobility. May be treated using a rigid orthotic with a high heel cup and medial and lateral flanges.
- Adult with rheumatoid arthritis. May be treated with an orthotic formed to the shape of a foot but made as a relatively soft device.

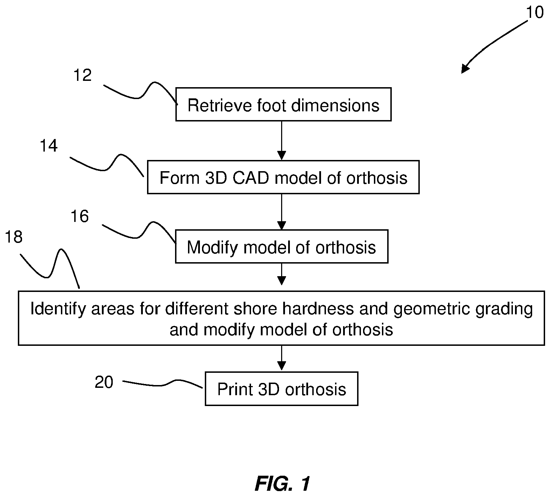

Referring to Figure 1, a flow diagram of a method of manufacturing a foot orthosis is indicated generally at 10.

The first step 12 in manufacturing the orthosis is to retrieve or obtain dimensions of a foot with which the orthosis is to be used. In this embodiment, the dimensions may be retrieved using a scan of the foot, for example using Delcam Orthomodel hardware and software (provided by Delcam pic, Small Heath Business Park, Birmingham, B10 0HJ, UK). In an alternative embodiment, the dimensions may be retrieved indirectly using an imprint of the foot. In a further alternative, an existing orthosis may be scanned in order to reproduce existing foot shape and corrective features. Software, and human input, is then used to form an initial design for the foot orthosis (again Delcam Orthomodel software can be used to do this). In an alternative embodiment, an orthosis designer may select an image of a foot or an orthosis that closely matches an intended user's foot or a suitable orthosis initial design from an image stock library.

Once an initial orthosis design has been established, the next step 14 is to import the image of the orthosis to an appropriate computer aided design (CAD) package. In this embodiment Rhino 3D software (provided by McNeel, 3670 Woodland Park Avenue, Seattle, WA 98103, USA) was used, but any appropriate CAD package may be used. The CAD package is used to appropriately manipulate the initial orthosis design. For example, the design may be thickened and edges may be capped off to form a solid surface model. The model may also be further manipulated to include other features required. For example, any extension portions to the foot orthosis, or any recesses or raised portions required.

In the next step 16, the model produced using the CAD package is imported to a haptic modelling software. In this embodiment Freeform® software (provided by Geomatic, Sensable Group, 181 Ballardvale Street, Wilmington, MA 01887, USA) was used. In the modelling software the model of the orthosis is shown on a computer screen with a visual representation of a shaping tool (e.g. a ball) on the end of a haptic arm connected to the computer is shown. The haptic arm includes a handheld device that can be held by a user in a similar manner to a pen. A designer can manipulate the handheld device so as to move the corresponding shaping tool on the screen in a manner that crops features of the orthosis model, adds features, and smooths and blends features of the orthosis. The haptic arm and software is capable of achieving a much improved smoothing and blending than that which can be achieved using a CAD package, and accordingly can improve comfort for a user of the resulting orthosis.

Once the model has been modified using the haptic arm and software the resulting 3D image file is exported from Freeform® as a mesh of triangular elements. The mesh is then checked to ensure the mesh is error free. A suitable software for this is Magics (provided by Materialise, Technologielaan 15, 3001 Leuven, Belgium). The size of the file may then be reduced (e.g. by triangle reduction) to decrease the load time while editing the model.

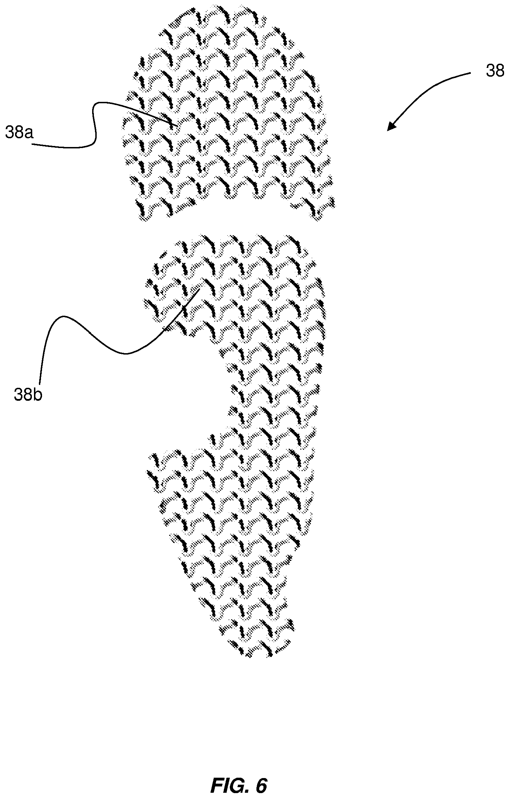

In the next step 18 of the method 10, the areas for increased support or compressibility are identified and the model of the orthosis is divided ("cut") into sections according to the level of support or compressibility required. The level of support or compressibility is determined using materials of a different shore hardness and/or geometric grading. The process of cutting the model into sections can be done using a CAD package, for example Rhino 3D. Figures 6 to 10 show examples of cut sections with different shore hardness and/or geometric grading, and will be explained in more detail later.

Rhino 3D is also used to form any geometric grading required.

The orthosis model is then exported to a software from which a 3D printer can operate. This will often be a software supplied with the relevant 3D printer. The software is then used to add data related to the shore hardness required in each of the cut sections based on the user's needs/patient's prescription to the model.

In the final step 20, the 3D printer is used to print the orthosis. A suitable 3D printer is Objet Connex 350 3D printer (provided by Stratasys, Ltd of Edina, Minnesota, USA - formerly Objet, 233 S. Detroit Ave, Suite 300 - Tulsa, OK 74120). The Connex 350 3D printer uses dual jets or outlets to enable simultaneous use of two materials for printing. The two materials can be mixed to provide the desired shore hardness (and other mechanical properties). Printing heads of the printer are used to apply thin layers of the desired material in the desired location in a build tray. Each layer applied is cured by UV light. The process is continued layer by layer until the final orthosis is formed. Materials of different shore hardness developed for printing using a specific 3D printer are used, these materials can often be sourced from the supplier of the printer. In this embodiment the orthosis is formed using material supplied from Objet (the 3D printer supplier), and the materials used have been developed specifically for 3D printing and UV curing.

If the insole comprises regions including geometric grading that includes one or more voids in the mass of material forming the orthosis, the voids are formed using a filler material that does not cure under UV light. Once the insole has been printed, the filler can be washed away to create the void.

The method described is for the production of a single orthosis. However, in alternative embodiments it is possible to modify the model inputted to the 3D printer software, so as to form multiple orthoses positioned side by side, in a single printing process.

Examples of orthoses formed using the above method, and options for geometric grading, will now be described.

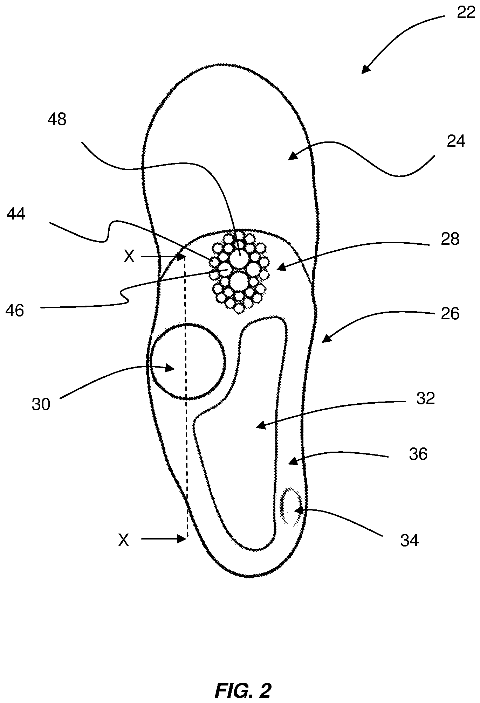

Referring to Figures 2 to 5, a foot orthosis is indicated generally at 22 and takes the general form of an insole or sock liner of a shoe, such that it can be easily positioned in an intended shoe. Figure 2 shows a side of the orthosis that is intended to be positioned nearest the sole of a foot of a user (i.e. the upperside of the orthosis). The side intended to be positioned nearest the sole of the foot of a user is formed of an extension portion 24 that is intended to be positioned nearest the toes of a user, and a support portion 26 intended to be positioned under the remainder of a user's foot.

The main support portion comprises a region 28 for supporting the arch of a user's foot; a region 30 for providing cushioning to a region of a user's foot (for example a relief region corresponding to a location where the user has a painful lump on the sole of their foot); a region 32 provides a strengthening region to support the orthosis; a region 34 defines a depression in the orthosis; and a region 36 forms a boundary of the support region 26 and is profiled at the edges to provide support for the sides of the foot and to more comfortably fit within a shoe.

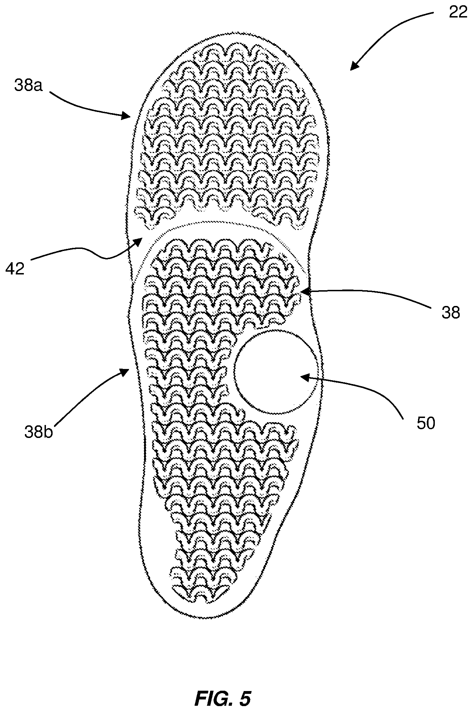

Referring to Figure 5, the side of the orthosis intended to be positioned nearest the sole of a shoe (i.e. the underside of the orthosis) is shown. In this embodiment, the orthosis has a curved rib structure 38 on its underside. The rib structure includes a plurality of wave-like ribs each extending across the width of the orthosis (i.e. corresponding to a direction from one side of the foot to the other, that is, transverse to a direction running from the heel of a foot to the toes of a foot). The ribs are aligned adjacent each other. The formation of the ribs enables the weight to be reduced, and reduces the amount of print material required which reduces the unit cost of the orthosis. A hole 50 extends through the thickness of the boundary region 36 to form a feature of the cushioned region 30 (as will be described later).

The ribs are formed in two sections, one section 38a positioned on the extension portion 24 of the orthosis and the other portion 38b positioned on the support portion 26 of the orthosis. The sections 38a and 38b of the rib structure 38 are shown separately in Figure 6, which shows the rib section as "cut" by the CAD package during manufacture.

A perimeter 42 extending around the underside of the orthosis is free from the rib structure 38.

Referring back to Figures 2 to 4, the regions of the upperside of the orthosis will now be described in more detail.

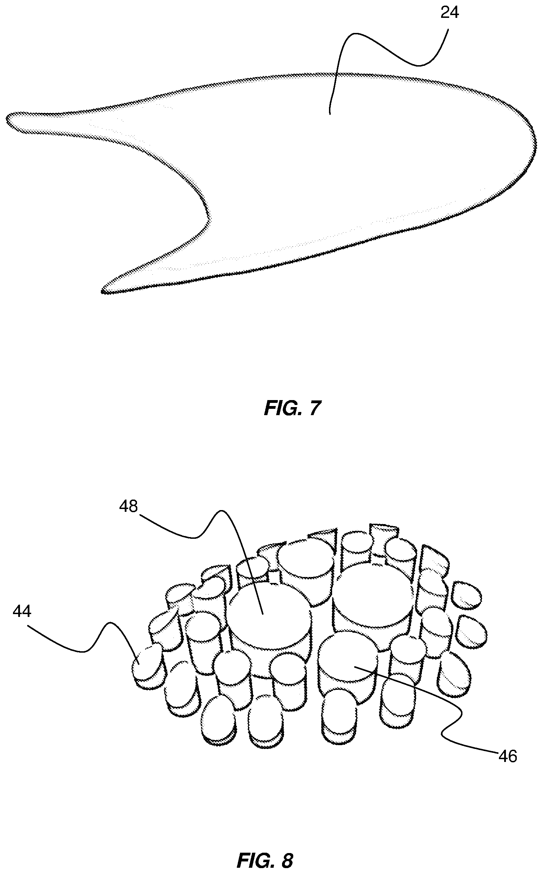

The region 24 is shown as a "cut" section (as it would be sectioned by the CAD package during the manufacturing process) in Figure 7. In this embodiment, the shore hardness selected is 60. All shore hardness measurements presented in this application are measured on the Shore A scale. The rib structure on the underside of the orthosis increases the flexibility of the orthosis, and the hardness of the region 24 is selected taking this reduction into account.

The region 28 is geometrically graded using a series of pillars provided in an oval arrangement. The pillars are arranged to be smaller on the perimeter of the region 28 and larger towards the centre of the region 28. In this embodiment there are three different sizes of pillars (both in height and in diameter). The smaller of the pillars 44 have a shore hardness of 60, the medium sized pillars 46 have a shore hardness of 70 and the largest pillars 48 have a shore hardness of 95. This arrangement of pillars of different sizes and shore hardness provides a graduation of hardness and compressibility from the perimeter of region 28 to the centre of region 28.

Figure 8 shows the "cut" section of pillars (separate from the orthosis), as would be "cut" using the CAD package during the manufacture of the orthosis. Such a region would be very difficult to achieve with prior art manufacturing techniques.

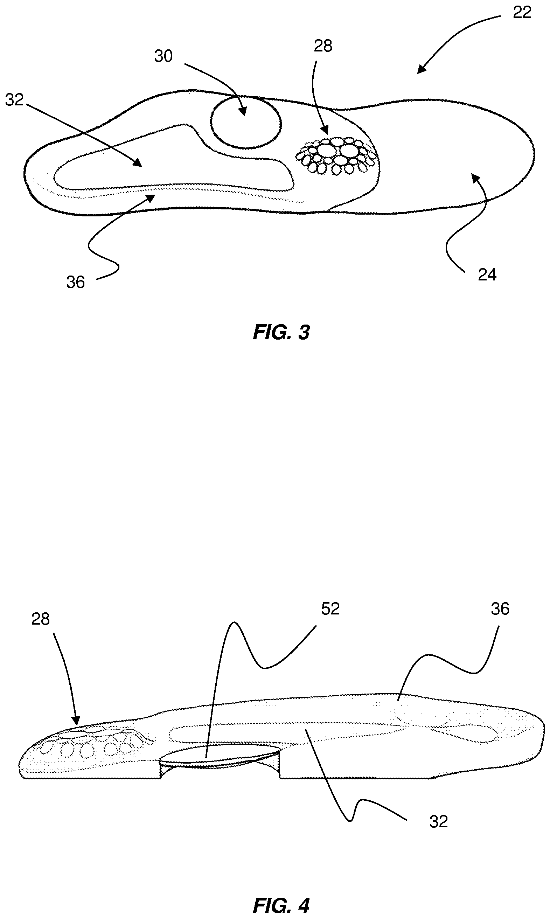

Region 30 is shown in more detail in the cross section shown in Figures 4. To form region 30, a hole 50 is formed through the boundary region 36. A surface structure 52 is formed integrally over the hole 50. The surface structure is circular in shape and dimensioned to correspond to the dimensions of the hole 50. The circumferential edge of the surface structure 52 is connected to the boundary region 36 at the edge of the hole on the upperside of the orthosis 22. In this embodiment, no structures are positioned within or beneath the hole so as to improve comfort for a user (i.e. if structures were positioned within the hole there is a risk that a user may feel the structures, and find the orthosis uncomfortable). In this embodiment the shore hardness selected for the surface structure 52 is 27.

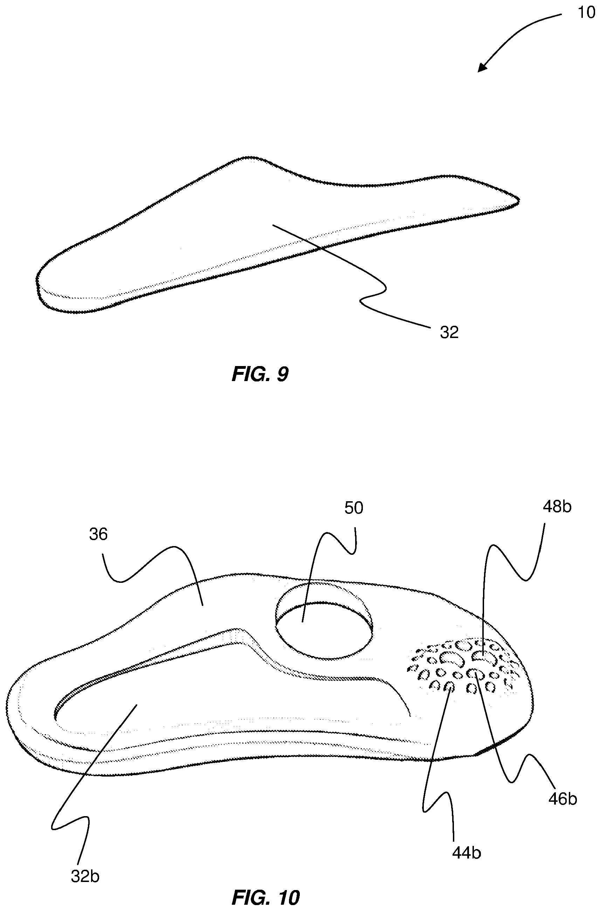

Referring back to Figures 2 to 4, region 32 is made from a high shore hardness to provide strength and stability to the orthosis. In this embodiment the shore hardness selected is 95, and this was selected taking into account the increase in flexibility provided by the rib structure 38 on the underside of the orthosis. The region as "cut" by the CAD package during manufacture is shown in Figure 9.

The region 34 is a depression formed in the boundary region 36.

Boundary region 36 is formed as a solid section of shore hardness 50. The hardness is selected to maintain flexibility at the edges of the orthosis so as to improve comfort. The boundary region 36 as sectioned using the CAD package during manufacture is shown in Figure 10. It can be seen that the boundary region 36 contains a hole 50, and recesses 32b, 44b, 46b, and 48b corresponding to the regions 30, 32, and pillars 44, 46 and 48 respectively. A depression is also provided corresponding to region 34, but this is not shown in Figure 10. As can be seen in an area of the boundary region corresponding to region 28, the boundary region is profiled to form a raised portion.

Although the regions have been described separately, they are all printed in a single print process so as to form the orthosis as an integrally formed component.

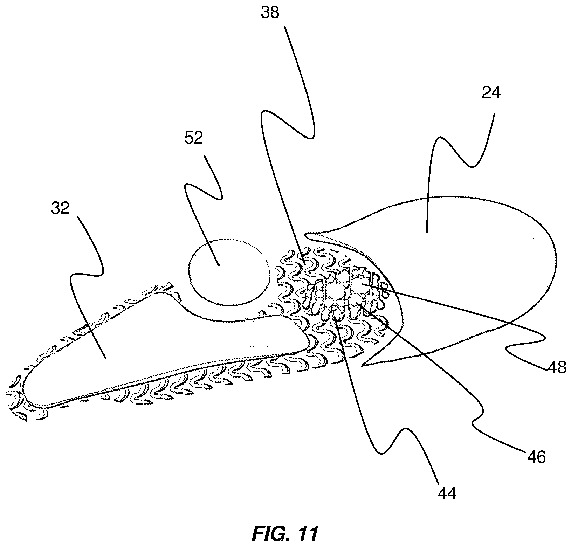

The arrangement of the regions without the boundary region 36 in place is shown in Figure 11.

The method of manufacture described is faster, less labour intensive and cheaper than the method of manufacture of the prior art. Furthermore, the process permits easier customisation of an orthosis to a user's needs. As has been illustrated with reference to the described orthosis, regions of the orthosis can be selected to have a different profile, different hardness and compressibility. The method of forming the foot orthosis permits the geometric grading and material hardness to be changed quickly and easily and allows for greater variation of material properties than the conventional construction method.

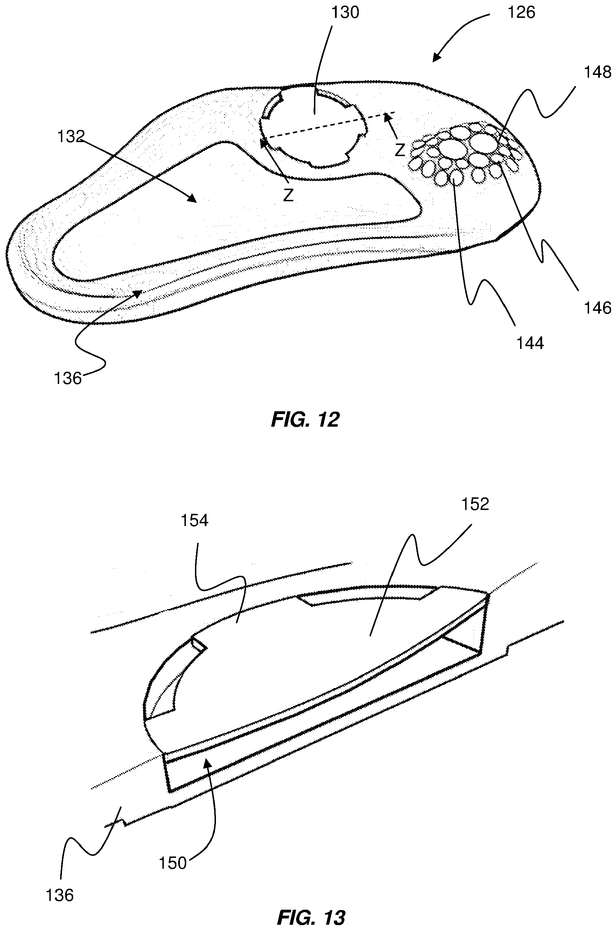

A support portion of an alternative orthosis is indicated generally at 126 in Figure 12. Many of the features are the same as the previously described embodiment and as such will not be described in detail again here. Similar reference numerals have been used to represent similar features, with the prefix "1" to distinguish between embodiments.

The main difference between the support structure 126 of Figure 12 and the previously described embodiment is the configuration of cushioned region 130, the structure of which is shown in more detail in Figure 13. Instead of the boundary region 136 comprising a hole as in the previously described embodiment, instead the boundary region 136 comprises a recess 152 (that does not extend through to the underside of the orthosis) formed in the vicinity of region 130. A surface structure 152 is positioned to cover the recess 150. The surface structure 152 is circular in shape and has four tabs 154 protruding around the perimeter thereof. The surface structure is dimensioned such that the tabs 154 connect the support surface 152 to an edge of the recess 150 on the upperside of the orthosis. In alternative embodiments the support structure may be modified to include two or three tabs, or to include five or more tabs.

Although the invention has been described above with reference to one or more preferred embodiments, it will be appreciated that various changes or modifications may be made without departing from the scope of the invention as defined in the appended claims. For example, it will be clear to the person skilled in the art that the shore hardnesses provided are by way of example only and the shore hardness of the regions, as well as the location and shape of each region, can be altered to meet the preferences of a user.

A foot orthosis with specific support and relief features has been described, but a variety of different support and/or relief features may be formed as part of the orthosis. For example, Kirby skives, met pads, plantar fascia grooves, dell, pronation skive, cobra pads, D pads, reverse mortons extention, medial additions, lateral additions, net dome, morton extension, PMP pad, full length extensions, forefoot posting, rear foot posting, pad support, insole covers, and/or cut-outs e.g. MPJ, 1^{st} Ray, u-shaped and/or wing shaped cut-outs.

In the described embodiment the orthosis was made to suit a user's specific requirements, but in alternative embodiments the method of manufacture may be used to produce "off-the- shelf' orthoses.

The examples of geometric grading provided include a surface structure, pillars and curved ribs. However, other geometric grading may be used, for example: strips of material aligned to linearly increase from a low shore hardness to a high shore hardness; wave forms used to support two opposing surfaces; domes; linear ribs; and any other suitable form of projection or recess, or any combination of said geometric grading features.

In further alternative embodiments the foot orthosis may have ventilation holes extending from the upperside to the underside of the orthosis.

In a yet further alternative embodiment the orthosis may take a shape suitable for insertion in a heeled shoe (e.g. a court shoe), or for insertion in any other type of specific shoe style, e.g. hiking boots or football boots.

In another alternative embodiment the foot orthosis may be manufactured so as to form the entire sole of a shoe (e.g. the insole, midsole, and outsole), instead of being designed to be positioned within a shoe. For example, the orthosis may be formed for connection to the thong of a flip-flop so as to form a flip-flop sole. In a further alternative embodiment the thong of the flip-flop may be formed during the 3D printing process.

## Claims

1. A method of manufacturing an orthosis (22) comprising:
providing electronic 3D data defining a shape of an orthosis (22) and a first region (26) and a second region (24) of the orthosis (22), the first region (26) having a greater compressibility than the second region (24); and
using a 3D printer to print an orthosis (22) based on the electronic 3D data,
**characterised in that**:
the 3D printer selectively uses materials of two or more shore hardnesses to print the orthosis (22) to provide varying hardness across the orthosis (22), said materials being printed together in a single 3D printing process, such that the first region (26) is integrally formed with the second region (24).

2. The method according to claim 1, wherein two materials of different shore hardnesses are mixed in varying quantities to provide varying shore hardness across the orthosis (22).

3. The method according to any one of the previous claims, wherein the first (26) and/or second region (24) comprises geometric grading for a greater compressibility in the first region (26) than in the second region (24).

4. The method according to claim 3, wherein the geometric grading utilises one or more of dome shapes, wave shapes, pillars (44, 46, 48), tensioned surfaces, cylinders and/or strips of material.

5. The method according to claim 4, wherein the first region (26) includes a surface structure (52) connected to the remainder of the orthosis (22), and wherein a region (50) below the surface structure (52) is hollow.

6. The method according to any one of the previous claims, wherein the 3D data defines a transition region (28) between the first region (26) and the second region (24), and the transition region (28) has graduated compressibility decreasing in compressibility from a position in the transition region (28) nearest the first region (26) to a position in the transition region (28) nearest the second region (24).

7. The method according to claim 6, wherein the transition region (28) is formed using geometric grading using materials of two or more shore hardnesses.

8. The method according to any one of the previous claims, wherein the 3D printer prints the orthosis (22) as a layered structure, such that a side of the orthosis (22) nearest the body of a user has a lower shore hardness than a side of the orthosis furthest from the body of a user.

9. The method according to any one of the previous claims, wherein the shape defined by the 3D data includes an indentation pattern arranged such that a side of the printed orthosis (22), intended to be positioned furthest from a body of a user, includes the indentation pattern.

10. The method according to any one of the previous claims, wherein the method comprises printing multiple orthoses (22) in a single printing process.

11. The method according to any one of the previous claims comprising a step of building a CAD model from which at least a portion of the 3D data is derived.

12. The method according to claim 11, comprising scanning a body part against which the orthosis (22) is to be positioned, and extracting dimensions therefrom to form the CAD image.

13. An orthosis (22) comprising a first region (26) and a second region (24), the first region (26) having greater compressibility than the second region (24),
**characterised in that**:
the first region (26) is integrally formed with the second region (24), wherein one or more materials of the first region (26) have a different shore hardness to one or more materials of the second region (24), said materials being printed together in a single 3D printing process.

14. The orthosis (22) according to claim 13, wherein a transition region (28) is provided between the first (26) and second (24) region, and the transition region (28) has graduated compressibility decreasing in compressibility from a position in the transition region (28) nearest the first region (26) to a position in the transition region (28) nearest the second region (24).

## Patentansprüche

1. Verfahren zum Herstellen einer Orthese (22), mit den Schritten:
Bereitstellen elektronischer 3D-Daten, die eine Form einer Orthese (22) und einen ersten Bereich (26) und einen zweiten Bereich (22) der Orthese definieren, wobei der erste Bereich (26) eine höhere Kompressibilität hat als der zweite Bereich (24); und
Verwenden eines 3D-Druckers zum Drucken einer Orthese (22) basierend auf den elektronischen 3D-Daten;
**dadurch gekennzeichnet, dass**
der 3D-Drucker selektiv Materialien mit zwei oder mehr Shore-Härten verwendet, um die Orthese (22) derart zu drucken, dass über die Orthese (22) verschiedene Härtegrade bereitgestellt werden, wobei die Materialien in einem einzelnen 3D-Druckvorgang zusammen gedruckt werden, so dass der erste Bereich (26) mit dem zweiten Bereich (24) integral ausgebildet wird.

2. Verfahren nach Anspruch 1, wobei zwei Materialien mit unterschiedlichen Shore-Härten in verschiedenen Mengen gemischt werden, um verschiedene Shore-Härten über die Orthese (22) bereitzustellen.

3. Verfahren nach einem der vorangehenden Ansprüche, wobei der erste Bereich (26) und/oder der zweite Bereich (24) eine geometrische Stufung aufweist, um im ersten Bereich (26) eine größere Kompressibilität zu erhalten als im zweiten Bereich (24).

4. Verfahren nach Anspruch 3, wobei die geometrische Stufung eine oder mehrere Formen unter Kuppelformen, Wellenformen, Säulenformen (44, 46,48), gespannten Flächen, Zylindern und/oder Materialstreifen verwendet.

5. Verfahren nach Anspruch 4, wobei der erste Bereich (26) eine Oberflächenstruktur (52) aufweist, die mit dem Rest der Orthese (22) verbunden ist, und wobei ein Bereich (50) unterhalb der Oberflächenstruktur (52) hohl ist.

6. Verfahren nach einem der vorangehenden Ansprüche, wobei die 3D-Daten einen Übergangsbereich (28) zwischen dem ersten Bereich (26) und dem zweiten Bereich (24) definieren, und wobei der Übergangsbereich (28) eine abgestufte Kompressibilität aufweist, die von einer Position im Übergangsbereich (28), die dem ersten Bereich (26) am nächsten ist, zu einer Position im Übergangsbereich (28) abnimmt, die dem zweiten Bereich (24) am nächsten ist.

7. Verfahren nach Anspruch 6, wobei der Übergangsbereich (28) mittels einer geometrischen Stufung unter Verwendung von Materialien mit zwei oder mehr Shore-Härten ausgebildet wird.

8. Verfahren nach einem der vorangehenden Ansprüche, wobei der 3D-Drucker die Orthese (22) als eine Schichtstruktur druckt, so dass eine Seite der Orthese (22), die am nächsten zum Körper eines Benutzers angeordnet ist, eine geringere Shore-Härte hat als eine Seite der Orthese (22), die am weitesten vom Körper eines Benutzers weg angeordnet ist.

9. Verfahren nach einem der vorangehenden Ansprüche, wobei die durch die 3D-Daten definierte Form ein Vertiefungsmuster aufweist, das derart angeordnet ist, dass eine Seite der Orthese (22), die dafür vorgesehen ist, am weitesten vom Körper eines Benutzers weg angeordnet zu werden, das Vertiefungsmuster aufweist.

10. Verfahren nach einem der vorangehenden Ansprüche, wobei das Verfahren das Drucken mehrere Orthesen (22) in einem einzigen Druckvorgang aufweist.

11. Verfahren nach einem der vorangehenden Ansprüche, mit einem Schritt zum Erzeugen eines CAD-Modells, von dem mindestens ein Teil der 3D-Daten hergeleitet wird.

12. Verfahren nach Anspruch 11, mit dem Scannen eines Körperteils, an dem anliegend die Orthese (22) angeordnet werden soll, und Extrahieren von Abmessungen davon, um das CAD-Bild zu erzeugen.

13. Orthese (22) mit einem ersten Bereich (26) und einem zweiten Bereich (24), wobei der erste Bereich (26) eine größere Kompressibilität hat als der zweite Bereich (24):
**dadurch gekennzeichnet, dass**
der erste Bereich (26) mit dem zweiten Bereich (24) integral ausgebildet ist,
wobei ein oder mehrere Materialien des ersten Bereichs (26) eine andere Shore-Härte aufweisen als ein oder mehrere Materialien des zweiten Bereichs (24), wobei die Materialien in einem einzigen 3D-Druckvorgang zusammen gedruckt werden.

14. Orthese (22) nach Anspruch 13, wobei ein Übergangsbereich (28) zwischen dem ersten Bereich (26) und dem zweiten Bereich (24) bereitgestellt wird, und wobei der Übergangsbereich (28) eine abgestufte Kompressibilität aufweist, die von einer Position im Übergangsbereich (28), die am nächsten zum ersten Bereich (26) angeordnet ist, zu einer Position im Übergangsbereich (28) abnimmt, die am nächsten zum zweiten Bereich (24) angeordnet ist.

## Revendications

1. Procédé de fabrication d'une orthèse (22) comprenant :
de fournir des données 3D électroniques définissant une forme d'une orthèse (22) et une première région (26) et une seconde région (24) de l'orthèse (22), la première région (26) ayant une plus grande compressibilité que la seconde région (24) ; et
d'utiliser une imprimante 3D pour imprimer une orthèse (22) en fonctions des données 3D électroniques,
**caractérisé en ce que** :
l'imprimante 3D utilise sélectivement des matériaux de deux ou plusieurs duretés Shore pour imprimer l'orthèse (22) pour fournir une dureté variable sur l'orthèse (22), lesdits matériaux étant imprimés ensemble dans un seul processus d'impression 3D, de telle manière que la première région (26) est formée d'un seul bloc avec la seconde région (24).

2. Procédé selon la revendication 1, dans lequel deux matériaux de duretés Shore différentes sont mélangés en quantités variables pour fournir des duretés Shore différentes sur l'orthèse (22).

3. Procédé selon l'une quelconque des revendications précédentes, dans lequel la première (26) et ou la seconde région (24) comprennent des reliefs géométriques pour une plus grande compressibilité dans la première région (26) que dans la seconde région (24).

4. Procédé selon la revendication 3, dans lequel le relief géométrique utilise une ou plusieurs formes de dôme, formes d'onde, piliers (44, 46, 48), surfaces tendues, cylindres et/ou bandes de matériau.

5. Procédé selon la revendication 4, dans lequel la première région (26) inclut une structure de surface (52) reliée au reste de l'orthèse (22), et dans lequel une région (50) sous la structure de surface (52) est creuse.

6. Procédé selon l'une quelconque des revendications précédentes, dans lequel les données 3D définissent une région de transition (28) entre la première région (26) et la seconde région (24), et la région de transition (28) a une compressibilité progressive diminuant en compressibilité depuis une position dans la région de transition (28) la plus proche de la première région (26) jusqu'à une position dans la région de transition (28) la plus proche de la seconde région (24).

7. Procédé selon la revendication 6, dans lequel la région de transition (28) est formée en utilisant un relief géométrique utilisant des matériaux de deux ou plusieurs duretés Shore.

8. Procédé selon l'une quelconque des revendications précédentes, dans lequel l'imprimante 3D imprime l'orthèse (22) comme une structure en couches, de telle manière qu'un côté de l'orthèse (22) le plus proche du corps d'un utilisateur a une dureté Shore plus petite qu'un côté de l'orthèse le plus loin du corps d'un utilisateur.

9. Procédé selon l'une quelconque des revendications précédentes, dans lequel la forme définie par les données 3D inclut un motif de dentelures agencé de telle manière qu'un côté de l'orthèse (22) imprimée, destiné à être positionné le plus loin d'un corps d'un utilisateur, inclut le motif de dentelures.

10. Procédé selon l'une quelconque des revendications précédentes, dans lequel le procédé comprend d'imprimer de multiples orthèses (22) dans un seul processus d'impression.

11. Procédé selon l'une quelconque des revendications précédentes comprenant une étape de construction d'un modèle CAD à partir duquel au moins une partie des données 3D sont dérivées.

12. Procédé selon la revendication 11, comprenant de scanner une partie de corps contre laquelle l'orthèse (22) doit être positionnée, et d'en extraire des dimensions pour former l'image CAD.

13. Orthèse (22) comprenant une première région (26) et une seconde région (24), la première région (26) ayant une plus grande compressibilité que la seconde région (24),
**caractérisée en ce que** :
la première région (26) est formée d'un seul bloc avec la seconde région (24), dans laquelle un ou plusieurs matériaux de la première région (26) a une dureté Shore différente d'un ou plusieurs matériaux de la seconde région (24), lesdits matériaux étant imprimés ensemble dans un seul processus d'impression 3D.

14. Orthèse (22) selon la revendication 13, dans laquelle une région de transition (28) est fournie entre la première (26) et la seconde (24) région, et la région de transition (28) a une compressibilité diminuant en compressibilité depuis une position dans la région de transition (28) la plus proche de la première région (26) jusqu'à une position dans la région de transition (28) la plus proche de la seconde région (24).
